# EUROPEAN PATENT SPECIFICATION

(11) **EP 3 463 331 B1**
(45) Date of publication and mention of the grant of the patent: **13.12.2023**
(21) Application number: 17734380.3
(22) Date of filing: 06.06.2017
(51) Int. Cl.: A61K 31/198, A61K 31/375, A61K 31/455, A61P 25/06, A61K 45/06

(54) **COMBINATION OF L-CYSTEINE, ASCORBIC ACID AND VITAMIN B3 FOR PREVENTING AND/OR DIMINISHING SYMPTOMS OF HANGOVER**
KOMBINATION VON L-CYSTEIN, ASCORBINSÄURE UND VITAMIN B3 ZUR VERHINDERUNG UND/ODER VERMINDERUNG VON SYMPTOMEN EINES KATERS
COMBINAISON DE L-CYSTÉINE, D'ACIDE ASCORBIQUE ET DE VITAMINE B3 POUR PRÉVENIR ET/OU RÉDUIRE LES SYMPTÔMES D'UNE VEISALGIE

(30) Priority: 06.06.2016 FI 20165468
(43) Date of publication of application: 10.04.2019
(73) Proprietor: Hokka, Pekka, 02180 Espoo (FI); Kolari, Juha, 05880 Hyvinkää (FI); Leino, Mikko, 07110 Hinthaara (FI)
(72) Inventor: HOKKA, Pekka, 02180 Espoo (FI); KOLARI, Juha, 05880 Hyvinkää (FI); LEINO, Mikko, 07110 Hinthaara (FI); POHJALAINEN, Timo, deceased (FI)
(74) Representative: Kolster Oy Ab
(86) International application number: PCT/FI2017/050417
(87) International publication number: WO 2017/212113

(56) References cited:
- WO-A2-2012/027603
- DE-A1-102011 013 224
- US-B1- 8 440 241

## Description

### Field of the invention

The present invention relates to a composition for preventing and/or diminishing symptoms of hangover caused by alcohol consumption in humans. The invention relates also to use of the composition for preventing and/or diminishing the symptoms of hangover caused by alcohol consumption in humans.

### Background of the invention

It is known that the consumption of alcohol may cause several unpleasant symptoms such as hangover in humans. The symptoms of hangover include physiological and mental symptoms. The physiological symptoms comprise headache, fatigue, nausea, dizziness, thirst, anxiety, tremor and perspiration, for example. The mental symptoms comprise drinker's depression and anxiety, for example. The types and intensities of the symptoms vary within the individuals. Prevention and/or reduction of hangover symptoms would be advantageous not only from the point of view of the individual suffering from the hangover symptoms but also from the one of the society in general.

Alcohol is metabolized by several processes or pathways. The most common of these pathways involves two enzymes-alcohol dehydrogenase and aldehyde dehydrogenase. These enzymes help break apart the alcohol molecule, making it possible to eliminate it from the body. First, alcohol dehydrogenase metabolizes alcohol to acetaldehyde, a highly toxic substance. Then, in a second step, acetaldehyde is further metabolized down to acetate which then is broken down into water and carbon dioxide.

Regardless of how much a person consumes, the body can only metabolize a certain amount of alcohol every hour. That amount varies widely among individuals and depends on a range of factors, including liver size and body mass. Some of the alcohol metabolizing enzymes work more or less efficiently than others; this means that some people can break down alcohol to acetaldehyde, or acetaldehyde to acetate, more quickly than others, there may also be differences in the alcohol metabolism due to the genetic background. A fast alcohol dehydrogenase enzyme or a slow aldehyde dehydrogenase enzyme can cause toxic acetaldehyde to build up in the body, creating dangerous and unpleasant hangover effects that also may affect an individual's risk for various alcohol-related problems-such as developing alcoholism.

Compositions for alleviating symptoms caused by alcohol consumption are known in the art. WO 2016142580 A1 discloses an oral composition for preventing the development of hangover symptoms. US 2007202215 A1 discloses a nutritional supplement for humans who consume moderate to large amounts of alcohol. The supplement is designed to compensate for malnutrition and toxic effects caused by long-term alcohol consumption. WO 2013072441 A1 discloses compositions for minimizing the cell-damaging effect of radicals and harmful forms of oxygen. It is noted in WO 2013072441 A1 that ingestion of acetaldehyde or consumption of alcohol may cause formation of radicals. WO 2012027603 A2 discloses compositions that provide nutrients for improving production of enzymes that are reduced in the body because of alcohol consumption. Especially aldehyde dehydrogenase (ALDH2) enzyme deficiency is targeted. EP 1909600 B1 discloses a composition for supporting and/or moderating the alcohol degradation process within the human body and reducing the alcohol induced risk for various diseases. CN 101766637 B discloses a composition for use as a healthcare food and for relieving alcohol consumption related symptoms including hangover.

Compositions known in the art comprise ingredients such as carbohydrates that are easily digested and metabolized and contain energy. Furthermore, several compositions known in the art comprise ingredients such as herbal and probiotic preparations that cause non predictable effects. Therefore, further solutions are needed to address the problem of preventing and/or diminishing symptoms of hangover caused by ethanol consumption in humans.

### Brief description of the invention

An object of the present invention is to provide reduction in the toxic effects and/or physiological symptoms caused by the consumption of alcohol in a human. In particular, an object of the present invention is to prevent and/or reduce the physiological symptoms of hangover, such as headache, fatigue, nausea, dizziness, thirst, anxiety, tremor and perspiration and ease hangover after alcohol consumption. In addition, an object of the present invention is to prevent and/or reduce the mental symptoms of hangover, such as drinker's depression and anxiety.

The objects of the invention are achieved by compositions, which are characterized by what is stated in the independent claims. The preferred embodiments of the invention are disclosed in the dependent claims.

The invention is based on the realization that a composition consisting L-cysteine, ascorbic acid and vitamin B3 as the active ingredients was found to reduce the physiological and mental symptoms caused by the consumption of alcohol in a human. It was also realized that the hangover effects caused by toxic acetaldehyde need to be prevented already during the alcohol consumption.

An advantage of the present invention is that the need for medicating symptoms of hangover with drugs having toxic side effects is diminished or even abolished.

A further advantage of the present invention is that the composition consist both L-cysteine and ascorbic acid (vitamin C). These active ingredients function in a synergistic manner to protect cells from damage induced by alcohol consumption. More precisely, ascorbic acid enhances the binding of L-cysteine to acetaldehyde, a metabolite of alcohol.

A further advantage of the present invention is that the composition is defined, comprising essential vitamins and a conditionally essential amino acid. Nonessential nutrients or components with non-defined composition such as herbal and probiotic preparations are not included in the composition. Therefore, the effects of the composition are more easily predicted. For example persons with allergy, hypersensitivity or intolerance symptoms can easily determine suitability of the composition for their use.

A further advantage of the present invention is that the composition does not comprise carbohydrates such as sugars, oligosaccharides or starches that are readily digested and metabolized. The composition of the present invention therefore contains less energy and is more tooth friendly. In addition, the composition of the present invention is easily ingestible due to smaller dose volume caused by the lack of carbohydrates. Carbohydrates can increase metabolism of alcohol, leading to higher peak concentrations of acetaldehyde in the body.

A further advantage of the present invention is that the active ingredients begin dissolving directly upon ingestion. The composition comprises no additives that would cause a delay in the release of the active ingredients. Therefore, effects of the composition such as the binding reaction between L-cysteine and acetaldehyde commence immediately after ingestion of the composition.

A further advantage of the present invention is the timing of taking or administering the composition. The composition can be taken before, during or after consumption of alcohol, and one or several doses of the composition can be taken during the event of alcohol consumption. This facilitates the sizing of total dosage by the user. The sizing of dosage can be done according to, for example, the amount of alcohol consumed, the amount of time in which the alcohol is consumed and individual needs such as the severity of hangover symptoms typically experienced by the user.

A further advantage of the present invention is the prevention or alleviation of symptoms of alcohol withdrawal syndrome. Symptoms of alcohol withdrawal syndrome range from mild to severe and comprise anxiety, shakiness, sweating, nausea, vomiting, fast heart rate, mild fever, seizures, increased hand tremor, insomnia, transient hallucinations (auditory, visual or tactile), psychomotor agitation, tonic-clonic seizures and autonomic instability. The symptoms of alcohol withdrawal syndrome have many similarities with symptoms of hangover and can be alleviated or prevented by taking the composition of the present invention.

A further advantage of the present invention is the prevention or delay of onset of alcoholism. Persons with less severe or absent symptoms of hangover are less likely to again start consuming alcohol to prevent or delay the onset of hangover. Therefore, habitual consumption of alcohol is less likely to develop without recurring consumption, i.e. a morning after pick-me-up.

### Detailed description of the invention

The present invention relates to a composition for preventing and/or diminishing symptoms of hangover caused by alcohol consumption in a human.

The term alcohol as used herein refers to the primary alcohol ethanol (ethyl alcohol) and especially to the alcoholic beverages.

Specifically the present invention relates to a composition consisting L-cysteine, ascorbic acid (vitamin C) and vitamin B3 (niacin) as the active ingredients. In addition, the composition of one or more additional active ingredient selected from vitamin B1 (thiamine), vitamin B2 (riboflavin), vitamin B6 (pyridoxine), vitamin B12 (cobalamin), folic acid and D-biotin. Further, the composition may comprise excipients and adjuvants acceptable for use in dietary supplements and/or pharmaceuticals.

The composition of the present invention consists of L-cysteine in an amount of about 60 to about 75 weight-%, ascorbic acid in an amount of about 20 to about 35 weight-% and vitamin B3 in an amount of about 4.0 to about 6.5 weight-%, based on the total weight of the composition. These active ingredients were found to reduce the physiological and mental symptoms caused by the consumption of alcohol.

L-cysteine is a conditionally essential amino acid. It is a precursor of antioxidants such as glutathione that is essential for the breakdown of toxins in the body, such as alcohol. Glutathione is a tripeptide molecule that prevents cellular damage caused by reactive oxygen species induced by e.g. alcohol consumption and also directly sequesters acetate to be excreted from the body with urine. Glutathione is metabolized in the liver, and its supply in the liver is largely depleted after consumption of two doses of alcohol, i.e. approximately 33 grams of ethyl alcohol. After this, accumulation of acetaldehyde can start causing symptoms such as hangover. In addition to acting as a precursor and therefore helping to maintain a supply of glutathione, L-cysteine also directly binds acetaldehyde.

In one embodiment, the composition consists of L-cysteine about 60 to about 75 weight-% based on the total weight of the active ingredients in the composition. In one embodiment, the composition consists of L-cysteine about 67 to about 75 weight-% based on the total weight of the active ingredients in the composition. In one embodiment, the composition consists of L-cysteine about 67 to about 70 weight-% based on the total weight of the active ingredients in the composition. In one embodiment, the composition consists of L-cysteine about 67.0 weight-% based on the total weight of the active ingredients in the composition.

Ascorbic acid or L-ascorbic acid is an essential nutrient in human diets. Its biologically active form, vitamin C, is an antioxidant and functions as a reducing agent and a coenzyme in metabolic pathways in a human. In the present invention ascorbic acid accelerates neutralization of acetaldehyde by enhancing binding of L-cysteine and glutathione to acetaldehyde already during consumption of alcohol. In addition, in the present invention the antioxidative effect of ascorbic acid can also protect from the aging effect of alcohol.

In one embodiment, the composition consists of ascorbic acid about 20 to about 35 weight-% based on the total weight of the active ingredients in the composition. In one embodiment, the composition consists of ascorbic acid about 20 to about 25 weight-% based on the total weight of the active ingredients in the composition In one embodiment, the composition consists of ascorbic acid about 25 to about 35 weight-% based on the total weight of the active ingredients in the composition. In one embodiment, the composition consists of ascorbic acid about 27.0 weight-%, based on the total weight of the active ingredients in the composition.

Metabolism of alcohol consumes a variety of B vitamins already during consumption of alcohol. B vitamins are important cofactors in the synthesis of glutathione and enhance neutralization of acetaldehyde. The body is depleted especially of vitamin B1 (thiamin) and vitamin B6 (pyridoxin) during alcohol metabolism, and vitamins B2 (riboflavin), B3 (niacin) and B9 (folic acid) are also consumed. Particularly important is also vitamin B3 (niacin), which is a water soluble vitamin functioning as a precursor for the coenzymes nicotinamide adenine dinucleotide (NAD) and nicotinamide adenine dinucleotide phosphate (NADP). NAD is important in catabolism of alcohol, for example.

In one embodiment, the composition consists of vitamin B3 about 4.0 to about 6.5 weight-%, based on the total weight of the active ingredients in the composition. In one embodiment, the composition consists of vitamin B3 about 4.0 to about 6.0 weight-%, based on the total weight of the active ingredients in the composition. In one embodiment, the composition consists of vitamin B3 about 4.5 to about 6.5 weight-%, based on the total weight of the active ingredients in the composition. In one embodiment, the composition vitamin B3 about 5.3 weight-%, based on the total weight of the active ingredients in the composition.

Supplementing the body also with vitamins B1, B2 and B6 in conjunction with alcohol ingestion can prevent hangover symptoms such as anxiety, restlessness and malaise.

According to the invention, the composition for preventing and/or diminishing symptoms of hangover caused by alcohol consumption in a human consists of L-cysteine about 60 to about 75 weight-%, ascorbic acid about 20 to about 35 weight-% and vitamin B3 about 4.0 to about 6.5 weight-%, based on the total weight of the active ingredients in the composition.

In one embodiment, the composition consists of L-cysteine about 67.0 weight-%, ascorbic acid about 27.0 weight-% and vitamin B3 about 6.0 weight-%, based on the total weight of the active ingredients in the composition. In one embodiment, the composition consists of L-cysteine about 67.0 weight-%, ascorbic acid about 27.0 weight-% and vitamin B3 about 5.3 weight-%, based on the total weight of the active ingredients in the composition.

The composition further consists of one or more additional active ingredient selected from vitamin B1, vitamin B2, vitamin B6, vitamin B12, folic acid and D-biotin.

In one embodiment, the composition comprises vitamin B1 in an amount about 0.15 to 0.30% based on the total weight of the composition. In one embodiment, the composition comprises vitamin B1 in an amount about 0.20 to 0.30% based on the total weight of the composition. In one embodiment, the composition comprises vitamin B1 in an amount about 0.22% based on the total weight the composition.

In one embodiment, the composition comprises vitamin B1 in an amount about 0.30 to 0.40% based on the total weight of the active ingredients in the composition. In one embodiment, the composition comprises vitamin B1 in an amount about 0.35 to 0.40% based on the total weight of the active ingredients in the composition. In one embodiment, the composition comprises vitamin B1 in an amount about 0.37% based on the total weight of the active ingredients in the composition.

In one embodiment, the composition comprises vitamin B2 in an amount about 0.25 to 0.35% based on the total weight of the composition. In one embodiment, the composition comprises vitamin B2 in an amount about 0.25 to 0.30% based on the total weight of the composition. In one embodiment, the composition comprises vitamin B2 in an amount about 0.28% based on the total weight of the composition.

In one embodiment, the composition comprises vitamin B2 in an amount about 0.35 to 0.50% based on the total weight of the active ingredients in the composition. In one embodiment, the composition comprises vitamin B2 in an amount about 0.40 to 0.50% based on the total weight of the active ingredients in the composition. In one embodiment, the composition comprises vitamin B2 in an amount about 0.47% based on the total weight of the active ingredients in the composition.

In one embodiment, the composition comprises vitamin B6 in an amount about 0.25 to 0.35% based on the total weight of the composition. In one embodiment, the composition comprises vitamin B2 in an amount about 0.25 to 0.30% based on the total weight of the composition. In one embodiment, the composition comprises vitamin B6 in an amount about 0.28% based on the total weight of the composition.

In one embodiment, the composition comprises vitamin B6 in an amount about 0.35 to 0.50% based on the total weight of the active ingredients in the composition. In one embodiment, the composition comprises vitamin B6 in an amount about 0.40 to 0.50% based on the total weight of the active ingredients in the composition. In one embodiment, the composition comprises vitamin B6 in an amount about 0.47% based on the total weight of the active ingredients in the composition.

In one embodiment, the composition comprises vitamin B12 in an amount about 0.0003 to about 0.0008% based on the total weight of the composition. In one embodiment, the composition comprises vitamin B12 in an amount about 0.0005% based on the total weight of the composition.

In one embodiment, the composition comprises vitamin B12 in an amount about 0.0005 to about 0.001% based on the total weight of the active ingredients in the composition. In one embodiment, the composition comprises vitamin B12 in an amount about 0.0008% based on the total weight of the active ingredients in the composition.

In one embodiment, the composition comprises folic acid in an amount about 0.03 to 0.06% based on the total weight of the composition. In one embodiment, the composition comprises folic acid in an amount about 0.04% based on the total weight of the composition.

In one embodiment, the composition comprises folic acid in an amount about 0.05 to about 0.10% based on the total weight of the active ingredients in the composition. In one embodiment, the composition comprises folic acid in an amount about 0.07% based on the total weight of the active ingredients in the composition.

In one embodiment, the composition comprises D-biotin in an amount about 0.007 to about 0.02% based on the total weight of the composition. In one embodiment, the composition comprises D-biotin in an amount about 0.01% based on the total weight of the composition.

In one embodiment, the composition comprises D-biotin in an amount about 0.01 to about 0.02% based on the total weight of the active ingredients in the composition. In one embodiment, the composition comprises D-biotin in an amount about 0.017% based on the total weight of the active ingredients in the composition.

In one embodiment, the composition comprises vitamin B1, vitamin B2, vitamin B6, vitamin B12, folic acid and D-biotin as additional active ingredients.

In one embodiment, the composition comprises L-cysteine, ascorbic acid, vitamin B3, vitamin B1, vitamin B2, vitamin B6, vitamin B12, folic acid and D-biotin as active ingredients.

In one embodiment, the composition consists of L-cysteine about 60 to about 75 weight-%, ascorbic acid about 20 to about 35 weight-% and vitamin B3 about 4.0 to about 6.5 weight-%, based on the total weight of the active ingredients in the composition, as well as vitamin B1, vitamin B2, vitamin B6, vitamin B12, folic acid and D-biotin as additional active ingredients. In one embodiment, the composition consists of L-cysteine about 60 to about 75 weight-%, ascorbic acid about 20 to about 35 weight% and vitamin B3 about 4.0 to about 6.5 weight-%, based on the total weight of the active ingredients in the composition, as well as vitamin B1, vitamin B2, vitamin B6, vitamin B12, folic acid and D-biotin as additional active ingredients in a total amount of about 1.0 to about 1.6 weight-%, based on the total weight of the active ingredients in the composition. In one embodiment, vitamin B1, vitamin B2, vitamin B6, vitamin B12, folic acid and D-biotin are included in a total amount of about 1.2 to 1.5 weight-%, based on the total weight of the active ingredients in the composition. In one embodiment, vitamin B1, vitamin B2, vitamin B6, vitamin B12, folic acid and D-biotin are included in a total amount of about 1.4 weight-%, based on the total weight of the active ingredients in the composition.

In one embodiment, the active ingredients of the composition consist of L-cysteine, ascorbic acid, vitamin B3, vitamin B1, vitamin B2, vitamin B6, vitamin B12, folic acid and D-biotin.

According to Commission Directive 200/100/EC, the recommended daily allowances for vitamin C, vitamin B1, vitamin B2, vitamin B3, vitamin B6, vitamin B12, folic acid and D-biotin are 80 mg, 1.1 mg, 1.4 mg, 16 mg, 1.4 mg, 2.5 µg, 200µg and 50 µg, respectively.

The terms "L-cysteine", "ascorbic acid", "vitamin B3", "vitamin B1", "vitamin B2", "vitamin B6", "vitamin B12", "folic acid" and "D-biotin" as used in the present specification and claims refer and cover the different chemical forms of these compounds (vitamers).

The composition of the present invention may be formulated to any dosage form suitable for oral administration to a human. In one embodiment, the composition is formulated into a solid dosage form such as a tablet or a granule. In one embodiment, the composition is in the form of a tablet. In one embodiment, the total weight of a tablet is in the range of 200 to 600 mg. In one embodiment, the total weight is in the range of 450 to 550 mg. In one embodiment, the weight of the tablet is 500 mg. In addition to the active ingredients, the composition of the present invention comprises excipients and adjuvants acceptable for use in dietary supplements and/or pharmaceuticals. Such excipients and adjuvants include bulking agents, anti-caking agents, carrier agents and flavors, for example. In one embodiment, the composition is formulated into a liquid form. In one embodiment, the composition is an aqueous solution. In addition to the active ingredients, the liquid composition of the present invention comprises excipients and adjuvants acceptable for use in liquid dietary supplements and/or pharmaceuticals such as water, buffering agents and flavors, for example.

Preferably the composition of the invention is in the form of a tablet and it contains at least 200 mg L-cysteine, at least 16.0 mg Vitamin B3 and at least 80.0 mg Ascorbic acid. Additionally it may contain other B vitamins.

In one embodiment, the composition of the present invention contains the following active ingredients: 200 mg L-cysteine, 1.10 mg Vitamin B1, 1.40 mg Vitamin B2, 16.0 mg Vitamin B3, 1.40 mg Vitamin B6, 0.0025 mg Vitamin B12, 0.20 mg Folic acid, 0.05 mg D-biotin and 80.0 mg Ascorbic acid together with microcrystalline cellulose, silicon dioxide, magnesium stearate and flavor and color compounds and is formulated into tablets.

The composition is used for preventing and/or diminishing the symptoms of hangover caused by alcohol consumption in humans. In one embodiment, the composition is for use in treating the symptoms of hangover caused by alcohol consumption in humans. In one embodiment, the composition is for use in preventing the symptoms of hangover caused by alcohol consumption in humans. In one embodiment, the composition is for use in diminishing the symptoms of hangover caused by alcohol consumption in humans. In one embodiment, the invention relates to a use of the composition for preventing and/or diminishing symptoms of hangover caused by alcohol consumption in humans. In one embodiment, the invention relates to a method for preventing and/or diminishing symptoms of hangover caused by alcohol consumption in humans by administering the composition to a subject.

The composition of the present invention can be administered before, during and/or even after the alcohol consumption. The administration may be designed based on the amount of alcohol consumed as well as the physiological and ethnic characteristics of the individual. The composition may be administered before, during and after the alcohol consumption. The composition may be administered sequentially within two, three or four hours, for example, after the previous administration. In one embodiment, the composition is administered before the alcohol consumption. In one embodiment, the composition is administered during the alcohol consumption. In one embodiment, the composition is administered right after the alcohol consumption. In one embodiment, the composition is administered after the alcohol consumption. In one embodiment, the composition is administered before and during the alcohol consumption. In one embodiment, the composition is administered before and after the alcohol consumption. In one embodiment, the composition is administered during and after the alcohol consumption. In one embodiment, the composition is administered before, during and after the alcohol consumption.

In one embodiment, the composition is administered as follows: one preparation is taken before alcohol consumption and one preparation is taken every one to two hours during alcohol consumption. In one embodiment, one preparation is taken once an hour starting from the beginning of consumption of alcohol.

The physiological and/or mental symptoms of hangover are reduced significantly by the administration of the composition according to the present invention. Without wishing to be bound by any theory, the inventors believe that the active components of the present composition facilitate the decrease of the level of acetaldehyde formed from alcohol in the body during and after alcohol drinking.

The composition of the present invention can prevent or alleviate the symptoms of alcohol withdrawal syndrome if taken during the alcohol consumption. Symptoms of alcohol withdrawal syndrome range from mild to severe and comprise anxiety, shakiness, sweating, nausea, vomiting, fast heart rate, mild fever, seizures, increased hand tremor, insomnia, transient hallucinations (auditory, visual or tactile), psychomotor agitation, tonic-clonic seizures and autonomic instability.

The composition and use of the present invention may also prevent or delay the onset of alcoholism. Persons with less severe or absent symptoms of hangover are less likely to again start consuming alcohol for preventing or delaying the onset of hangover. Therefore, habitual consumption of alcohol is less likely to develop without recurring consumption, i.e. a morning after pick-me-up.

### Examples

### Example 1

The composition of the present invention was prepared by mixing the following active ingredients:

| | |
|---|---|
| L-cysteine | 200.0 mg |
| Vitamin B1 | 1.10 mg |
| Vitamin B2 | 1.40 mg |
| Vitamin B3 | 16.0 mg |
| Vitamin B6 | 1.40 mg |
| Vitamin B12 | 0.0025 mg |
| Folic acid | 0.20 mg |
| D-biotin | 0.05 mg |
| Ascorbic acid | 80.0 mg |

together with microcrystalline cellulose, silicon dioxide, magnesium stearate and flavor and color compounds and formulated into tablets having total weight of 500 mg.

### Example 2

Six test persons consumed each 334 ml ethanol in the form of beer (4.5% alcohol), long drink (5.5% alcohol) and Koskenkorva, clear spirit drink (38% alcohol) over about 5 hour time period. The tablet composition disclosed in Example 1, was administered to four of the test persons before starting the alcohol consumption, two hours after the administration of the first tablet and four hours after the administration of the first tablet. Two of the test persons did not take the tablets. The consumed amount of alcohol would typically cause hangover symptoms including headache, nausea and drinker's depression, for example, afterwards to the test persons. Two of the test persons taking the tablets suffered no hangover symptoms and two of them suffered substantially reduced symptoms. The two test persons, who did not take the tablets, suffered in a typical manner from the hangover symptoms including headache, nausea and drinker's depression.

### Example 3

A test person, a 43 years old European male, consumed 334 ml ethanol in the form of beer (4.5% alcohol), long drink (5.5% alcohol) and Koskenkorva, clear spirit drink (38% alcohol) over about 5 hour time period. The test person suffered the hangover symptoms including headache, nausea and drinker's depression in a typical manner.

A week later having had the same liquation and nutrition as week before said test person consumed 334 ml ethanol in the form of beer (4.5% alcohol), long drink (5.5% alcohol) and Koskenkorva, clear spirit drink (38% alcohol) over about 5 hour time period. The tablet composition disclosed in Example 1, was administered to the test person before starting the alcohol consumption, two hours after the administration of the first tablet and four hours after the administration of the first tablet. The test person suffered no hangover symptoms.

### Example 4

Four test persons consumed 7 bottles of sparkling wine (12% alcohol) over about 6 hours time period. Two persons took the tablet disclosed in Example 1 every hour and all together 6 tablets were taken per person during the consumption of the wine. The first tablet was taken when the alcohol consumption started. The two other test persons took one tablet every 2 hours and 3 tablets were taken all together per person. The test persons who took the tablets every hour did not suffer hangover symptoms. The two test persons who took 3 tablets during the 7 hour period suffered some symptoms of hangover including headache and fatigue, but the symptoms were clearly reduced compared to the test persons earlier experience of hangover.

It will be obvious to a person skilled in the art that, as the technology advances, the inventive concept can be implemented in various ways. The invention and its embodiments are not limited to the examples described above but may vary within the scope of the claims.

## Claims

1. A composition for preventing and/or diminishing symptoms of hangover caused by alcohol consumption in a human, wherein the active ingredients of the composition consist of
a) L-cysteine about 60 to about 75 weight-%,
b) ascorbic acid about 20 to about 35 weight-%, and
c) vitamin B3 about 4.0 to about 6.5 weight-%,
based on the total weight of the active ingredients of the composition, wherein the composition further consists of one or more additional active ingredient(s) selected from vitamin B1, vitamin B2, vitamin B6, vitamin B12, folic acid and D-biotin.

2. The composition according to claim 1, wherein active ingredients of the composition consist of
a) L-cysteine about 67 weight-%,
b) ascorbic acid about 27 weight-%, and
c) vitamin B3 about 5.3 weight-%
based on the total weight of the active ingredients of the composition.

3. The composition of any one of claims 1 to 2, wherein the composition is for oral administration.

4. The composition of claim 3, wherein the composition is in the form of tablets.

5. The composition of claim 4, wherein the tablet contains at least 200 mg L-cysteine, at least 16.0 mg Vitamin B3 and at least 80.0 mg Ascorbic acid.

6. The composition according to any one of claims 1 to 5 for use in preventing and/or diminishing the symptoms of hangover caused by alcohol consumption in a human.

7. The composition according to any one of claims 1 to 5 for use in preventing and/or delaying the onset of alcoholism in a human.

8. The composition according to any one of claims 1 to 5 for use in preventing and/or alleviating symptoms of alcohol withdrawal syndrome in a human.

9. The composition for use according to any of claims 6 to 8, wherein the composition is administered before consumption of alcohol.

10. The composition for use according to any of claims 6 to 8, wherein the composition is administered during the consumption of alcohol.

11. The composition for use according to any of claims 6 to 8, wherein the composition is administered after the consumption of alcohol.

12. The composition for use according to any of claims 6 to 8, wherein the composition is administered before and during the consumption of alcohol.

13. The composition for use according to any of claims 6 to 8, wherein the composition is administered as follows:
a) one preparation is taken before alcohol consumption and one preparation is taken every one to two hours during alcohol consumption; or
b) one preparation is taken once an hour starting from the beginning of consumption of alcohol.

14. The composition according to claim 5 for use in preventing and/or diminishing the symptoms of hangover caused by alcohol consumption in a human, wherein the tablet is administered every one to two hours during alcohol consumption.

## Patentansprüche

1. Zusammensetzung zur Verhinderung und/oder Verminderung von Symptomen eines Katers, der durch Alkoholkonsum verursacht wurde, bei einem Menschen, wobei die Wirkbestandteile der Zusammensetzung bestehen aus
a) L-Cystein, etwa 60 bis etwa 75 Gew.-%,
b) Ascorbinsäure, etwa 20 bis etwa 35 Gew.-%, und
c) Vitamin B3, etwa 4,0 bis etwa 6,5 Gew.-%,
bezogen auf das Gesamtgewicht der Wirkbestandteile der Zusammensetzung, wobei die Zusammensetzung ferner aus einem oder mehreren zusätzlichen Wirkbestandteilen besteht, die aus Vitamin B1, Vitamin B2, Vitamin B6, Vitamin B12, Folsäure und D-Biotin ausgewählt sind.

2. Zusammensetzung nach Anspruch 1, wobei Wirkbestandteile der Zusammensetzung bestehen aus
a) L-Cystein, etwa 67 Gew.-%,
b) Ascorbinsäure, etwa 27 Gew.-%, und
c) Vitamin B3, etwa 5,3 Gew.-%,
bezogen auf das Gesamtgewicht der Wirkbestandteile der Zusammensetzung.

3. Zusammensetzung nach einem der Ansprüche 1 bis 2, wobei die Zusammensetzung zur oralen Verabreichung ist.

4. Zusammensetzung nach Anspruch 3, wobei die Zusammensetzung in der Form von Tabletten ist.

5. Zusammensetzung nach Anspruch 4, wobei die Tablette mindestens 200 mg L-Cystein, mindestens 16,0 mg Vitamin B3 und mindestens 80,0 mg Ascorbinsäure enthält.

6. Zusammensetzung nach einem der Ansprüche 1 bis 5 zur Verwendung bei der Verhinderung und/oder Verminderung der Symptome eines Katers, der durch Alkoholkonsum verursacht wurde, bei einem Menschen.

7. Zusammensetzung nach einem der Ansprüche 1 bis 5 zur Verwendung bei der Verhinderung und/oder Verzögerung des Beginns von Alkoholismus bei einem Menschen.

8. Zusammensetzung nach einem der Ansprüche 1 bis 5 zur Verwendung bei der Verhinderung und/oder Linderung von Symptomen von Alkoholentzugssyndrom bei einem Menschen.

9. Zusammensetzung nach einem der Ansprüche 6 bis 8, wobei die Zusammensetzung vor einem Konsum von Alkohol verabreicht wird.

10. Zusammensetzung nach einem der Ansprüche 6 bis 8, wobei die Zusammensetzung während des Konsums von Alkohol verabreicht wird.

11. Zusammensetzung nach einem der Ansprüche 6 bis 8, wobei die Zusammensetzung nach dem Konsum von Alkohol verabreicht wird.

12. Zusammensetzung nach einem der Ansprüche 6 bis 8, wobei die Zusammensetzung vor und während des Konsums von Alkohol verabreicht wird.

13. Zusammensetzung nach einem der Ansprüche 6 bis 8, wobei die Zusammensetzung wie folgt verabreicht wird:
a) ein Präparat wird vor einem Alkoholkonsum eingenommen und ein Präparat wird alle ein bis zwei Stunden während eines Alkoholkonsums eingenommen; oder
b) ein Präparat wird einmal stündlich startend von dem Beginn eines Konsums von Alkohol eingenommen.

14. Zusammensetzung nach Anspruch 5 zur Verwendung bei der Verhinderung und/oder Verminderung der Symptome eines Katers, der durch Alkoholkonsum verursacht wurde, bei einem Menschen, wobei die Tablette alle ein bis zwei Stunden während eines Alkoholkonsums verabreicht wird.

## Revendications

1. Composition pour prévenir et/ou réduire les symptômes d'une veisalgie due à la consommation d'alcool chez un humain, dans laquelle les principes actifs de la composition consistent en
a) L-cystéine à raison d'environ 60 à environ 75 % en poids,
b) acide ascorbique à raison d'environ 20 à environ 35 % en poids, et
c) vitamine B3 à raison d'environ 4,0 à environ 6,5 % en poids,
par rapport au poids total des principes actifs de la composition, laquelle composition consiste en outre en un ou plusieurs principes actifs additionnels choisis parmi la vitamine B1, la vitamine B2, la vitamine B6, la vitamine B12, l'acide folique et la D-biotine.

2. Composition selon la revendication 1, dans laquelle les principes actifs de la composition consistent en
a) L-cystéine à raison d'environ 67 % en poids,
b) acide ascorbique à raison d'environ 27 % en poids, et
c) vitamine B3 à raison d'environ 5,3 % en poids,
par rapport au poids total des principes actifs de la composition.

3. Composition selon l'une quelconque des revendications 1 et 2, laquelle composition est destinée à être administrée par voie orale.

4. Composition selon la revendication 3, laquelle composition est sous la forme de comprimés.

5. Composition selon la revendication 4, dans laquelle le comprimé contient au moins 200 mg de L-cystéine, au moins 16,0 mg de vitamine B3 et au moins 80,0 mg d'acide ascorbique.

6. Composition selon l'une quelconque des revendications 1 à 5, pour une utilisation dans la prévention et/ou la réduction des symptômes d'une veisalgie due à la consommation d'alcool chez un humain.

7. Composition selon l'une quelconque des revendications 1 à 5, pour une utilisation dans la prévention et/ou le retard de l'apparition d'un alcoolisme chez un humain.

8. Composition selon l'une quelconque des revendications 1 à 5, pour une utilisation dans la prévention et/ou le soulagement des symptômes du syndrome de sevrage alcoolique chez un humain.

9. Composition pour une utilisation selon l'une quelconque des revendications 6 à 8, laquelle composition est administrée avant la consommation d'alcool.

10. Composition pour une utilisation selon l'une quelconque des revendications 6 à 8, laquelle composition est administrée pendant la consommation d'alcool.

11. Composition pour une utilisation selon l'une quelconque des revendications 6 à 8, laquelle composition est administrée après la consommation d'alcool.

12. Composition pour une utilisation selon l'une quelconque des revendications 6 à 8, laquelle composition est administrée avant et pendant la consommation d'alcool.

13. Composition pour une utilisation selon l'une quelconque des revendications 6 à 8, laquelle composition est administrée comme suit :
a) une préparation est prise avant la consommation d'alcool et une préparation est prise toutes les une à deux heures pendant la consommation d'alcool ; ou
b) une préparation est prise une fois par heure à partir du début de la consommation d'alcool.

14. Composition selon la revendication 5, pour une utilisation dans la prévention et/ou la réduction des symptômes d'une veisalgie due à la consommation d'alcool chez un humain, dans laquelle le comprimé est administré toutes les une à deux heures pendant la consommation d'alcool.
